Europäisches Patentamt

European Patent Office

Office européen des brevets

(19)

(11) **EP 0 697 401 B1**

(12) **FASCICULE DE BREVET EUROPEEN**

(45) Date de publication et mention
de la délivrance du brevet:
**28.10.1998 Bulletin 1998/44**

(51) Int Cl.⁶: **C07C 323/52**, C07C 319/02

(21) Numéro de dépôt: **95401875.0**

(22) Date de dépôt: **10.08.1995**

(54) **Synthèse d'esters d'acides mercaptocarboxyliques**

Synthese von Mercaptocarbonsäureestern

Synthesis of mercaptocarboxylic acid esters

(84) Etats contractants désignés:
**AT BE CH DE DK ES FR GB GR IE IT LI LU NL PT
SE**

(30) Priorité: **19.08.1994 FR 9410145**

(43) Date de publication de la demande:
**21.02.1996 Bulletin 1996/08**

(73) Titulaire: **ELF ATOCHEM S.A.**
**92800 Puteaux, Hauts-de-Seine (FR)**

(72) Inventeurs:
• **Labat, Yves**
**F-64000 Pau (FR)**
• **Muller, Jean-Pierre**
**F-64360 Monein (FR)**

(74) Mandataire: **Leboulenger, Jean et al**
**Elf Atochem S.A.,**
**Département Propriété Industrielle,**
**Cedex 42 - La Défense 10**
**92091 Paris la Défense (FR)**

(56) Documents cités:
**DE-B- 2 354 098**      **US-A- 2 262 686**

• **CHEMICAL ABSTRACTS, vol. 80, no. 11, 1974
Columbus, Ohio, US; abstract no. 59499z, &
JP-A-48 086 818 (KYORITSU KAKO CO LTD)**

**Description**

La présente invention concerne la préparation d'esters d'acides mercapto-carboxyliques et a plus particulièrement pour objet la synthèse des esters de formule générale :

$$HS-X-COOR \qquad (I)$$

dans laquelle X représente un radical alkylène, linéaire ou ramifié, contenant de 1 à 4 atomes de carbone et R un radical alkyle, linéaire ou ramifié, contenant de 1 à 18 atomes de carbone, de préférence 1 à 12.

Ces esters connus sont industriellement utilisés comme produits intermédiaires, notamment pour la fabrication de dérivés de l'étain utiles comme stabilisants à la chaleur des poly(chlorure de vinyle) et, dans le cas des esters d'alcools inférieurs (notamment R = méthyl), pour la fabrication de dérivés hétérocycliques (thiophéniques ou thiazoliques) utiles comme agents phytosanitaires.

La principale voie d'obtention des esters (I) est l'estérification d'un alcool ROH par un acide HS-X-COOH, lui-même obtenu par action d'un sulfhydrate sur l'acide chloro (ou bromo)carboxylique correspondant. Malgré son excellent rendement global, cette méthode présente l'inconvénient de générer une quantité considérable de rejets salins aqueux.

On sait que les esters d'acides mercapto-carboxyliques peuvent également être préparés par action d'un sulfhydrate alcalin sur un ester d'acide halogénocarboxylique. Cette méthode a notamment été utilisée par R.M. ACHESON et al. (J.Chem. Soc. 1961, p.650-660, en particulier p.656) pour préparer l'α-mercapto-α-méthylpropionate de méthyle par action du sulfhydrate de sodium sur l'α-bromo-α-méthylpropionate de méthyle dans le méthanol anhydre; le rendement est très faible (environ 36 %). Peuvent également être mentionnées les publications de brevets JP 48-86818, 63-10755 et 2-304061 qui opèrent toutes en présence d'eau, soit en milieu hydroalcoolique (JP 48-86818 et 63-10755), soit en milieu eau-toluène en présence d'un agent de transfert de phase (JP 2-304061).

Il a maintenant été trouvé que la présence, dans le milieu réactionnel, de quantités notables d'eau (autre que celle provenant éventuellement de la formation in situ du sulfhydrate) est défavorable à la sélectivité de la réaction et que cette sélectivité est en outre améliorée en opérant sous une pression élevée d'hydrogène sulfuré.

L'invention a donc pour objet un procédé de préparation des esters de formule (I) par action du sulfhydrate d'ammonium ou d'un sulfhydrate alcalin ou alcalino-terreux sur l'ester halogéno-carboxylique correspondant de formule :

$$Y-X-COOR \qquad (II)$$

dans laquelle R et X ont les mêmes significations que ci-dessus, et Y désigne un atome de chlore ou de brome, caractérisé en ce qu'on effectue la réaction en milieu alcoolique anhydre ou substantiellement anhydre et sous une pression d'hydrogène sulfuré au moins égale à 10 bars absolus.

L'expression "substantiellement anhydre" signifie ici que le milieu réactionnel ne contient pas d'eau autre que celle pouvant provenir de l'éventuelle formation in situ du sulfhydrate comme c'est le cas, par exemple, pour la formation du sulfhydrate de sodium selon la réaction :

$$NaOH + H_2S \rightarrow NaSH + H_2O$$

Comme alcool on peut utiliser un alcool inférieur en $C_1$ à $C_4$, mais on préfère travailler dans l'isopropanol et surtout dans le méthanol.

La réaction peut être effectuée à une température allant de 0 à 80°C, mais on préfère opérer à une température comprise entre 10 et 60°C.

La pression d'hydrogène sulfuré peut aller jusqu'à 30 bars absolus, mais est avantageusement comprise entre 10 et 20 bar absolus.

Bien que le procédé selon l'invention s'applique aussi aux esters bromocarboxyliques, on préfère partir des esters chloro-carboxyliques obtenus avec d'excellents rendements (environ 96 %) suivant la réaction d'estérification :

$$Cl-X-COOH + ROH \rightarrow Cl-X-COOR + H_2O$$

Selon la nature de l'ester (II) mis en oeuvre, sa concentration initiale dans le milieu réactionnel peut aller de 1 à 5 moles par litre d'alcool.

Comme sulfhydrate on peut utiliser un sulfhydrate alcalin ou alcalino-terreux tel que, par exemple, NaSH, KSH et Ca(SH)$_2$, mais on préfère employer le sulfhydrate d'ammonium qu'on peut former in situ sans production d'eau. Le rapport molaire sulfhydrate/ester (II) est généralement compris entre 1 et 2, mais pour obtenir une conversion complète de l'ester (II) il va de préférence de 1,1 à 1,5. Les valeurs indiquées pour ce rapport sont évidemment à diviser par deux lorsqu'on utilise un sulfhydrate bifonctionnel comme Ca(SH)$_2$.

Le procédé selon l'invention peut être mis en oeuvre en continu ou en discontinu (batch). Par rapport au procédé usuel de préparation des esters d'acides mercapto-carboxyliques par estérification d'un alcool ROH par un acide HS-X-COOH, il présente l'avantage, d'une part, de sous-produire environ deux fois moins de sel d'ammonium ou de sel alcalin ou alcalino-terreux et, d'autre part, de réduire considérablement la quantité de rejets aqueux salins. En effet, après neutralisation par un acide (de préférence HCl ou H$_2$SO$_4$) de l'excès de sulfhydrate dans le mélange réactionnel, les sels précipités peuvent être facilement séparés par filtration, avant ou après évaporation de l'alcool.

Dans les exemples suivant qui illustrent l'invention sans la limiter, les pourcentages indiqués sont exprimés en poids et les pressions d'H$_2$S en bars absolus.

**EXEMPLE 1**

Dans un autoclave en acier inoxydable, on a chargé 151 g d'une solution méthanolique d'ammoniac à 9,1 % (soit 0,808 mole de NH$_3$) puis on a introduit de l'hydrogène sulfuré jusqu'à une pression de 12 bars absolus. On a ensuite introduit en 40 minutes, à l'aide d'une pompe, 120 g (0,57 mole) de chloroacétate de 2-éthylhexyle en maintenant la température à 10±2°C. On a agité les réactifs pendant une heure, puis on a décomprimé l'autoclave.

L'analyse de la solution méthanolique a montré qu'elle contenait 49,3 % de thioglycolate de 2-éthylhexyle et 1,2 % de thiodiglycolate de 2-éthylhexyle, soit un rendement de 97,4 % en thioglycolate de 2-éthylhexyle.

Après neutralisation à l'aide d'acide chlorhydrique, la solution méthanolique a été chauffée sous vide pour évaporer le méthanol, puis filtrée pour séparer le chlorure d'ammonium précipité et enfin distillée. On a ainsi recueilli un thioglycolate de 2-éthylhexyle de pureté supérieure à 99 % et exempt d'acidité résiduaire.

**EXEMPLE 2**

Dans le même réacteur qu'à l'exemple 1, on a chargé 350 g d'une solution méthanolique de sulfhydrate de sodium à 8,5 % (soit 0,53 mole de NaSH), puis on a porté la pression à 10 bars par introduction d'hydrogène sulfuré.

En maintenant la température à 10°C on a ensuite introduit 39 g (0,36 mole) de chloroacétate de méthyle.

Après réaction et retour à la pression atmosphérique, on a obtenu une solution méthanolique contenant :

8,7 % de thioglycolate de méthyle
0,96 % de thiodiglycolate de méthyle
< 0,01 % de chloroacétate de méthyle

ce qui correspond à un rendement de 89 % en thioglycolate de méthyle.

**EXEMPLES 3 à 9**

Selon le même processus opératoire qu'aux exemples 1 et 2, on a réalisé sept essais de préparation du thioglycolate de 2-éthylhexyle en faisant varier la pression d'H$_2$S, la température et/ou le rapport molaire sulfhydrate/chloroacétate de 2-éthylhexyle.

Dans les exemples 3 et 4, donnés à titre comparatif, le méthanol a été remplacé par de l'eau totalement (exemple 3) ou pour moitié (exemple 4).

Les conditions opératoires et les résultats obtenus sont résumés dans le tableau suivant où R représente le radical -CH$_2$COOC$_8$H$_{17}$.

| EXEMPLE | Sulfhydrate | Pression d'$H_2S$ (bars) | Température (°C) | Rapport molaire sulfhydrate/RCl | Conversion (%) du RCl | Rendements (%) en composé : | | | |
|---|---|---|---|---|---|---|---|---|---|
| | | | | | | RSH | R-S-R | R-SS-R | ROH |
| 3(a) comparatif | NH$_4$SH | 1 | 40 | 1,5 | 39,5 | 14,3 | 15,4 | 9,1 | 0,7 |
| 4 (b) comparatif | NH$_4$SH | 15 | 25-35 | 1,1 | 99,99 | 80 | 10 | 5 (c) | 5 |
| 5 | NH$_4$SH | 12 | 40 | 1,1 | 100 | 91 | 8,4 | 0 | 0,6 |
| 6 | NH$_4$SH | 13 | 18 | 1,1 | 100 | 94 | 5,6 | 0 | 0,4 |
| 7 comparatif | NH$_4$SH | 8 | 18 | 1,1 | 100 | 86 | 13,6 | 0 | 0,4 |
| 8 comparatif | NaSH | 1 | 40 | 2 | 100 | 42 | 50,1 | 6,8 | 1,1 |
| 9 | NaSH | 10 | 10 | 1,1 | 100 | 93 | 6,2 | 0 | 0,8 |

(a) Essai effectué dans l'eau

(b) Essai effectué dans un mélange 50/50 d'eau et de méthanol

(c) Y compris un peu d'acide thioglycolique et de thioglycolate de méthyle

EP 0 697 401 B1

**Revendications**

1. Procédé de préparation d'un ester d'acide mercapto-carboxylique de formule :

$$HS\text{-}X\text{-}COOR \qquad\qquad (I)$$

dans laquelle X représente un radical alkylène, linéaire ou ramifié, contenant de 1 à 4 atomes de carbone et R un radical alkyle, linéaire ou ramifié, contenant de 1 à 18 atomes de carbone par action du sulfhydrate d'ammonium ou d'un sulfhydrate alcalin ou alcalino-terreux sur l'ester halogéno-carboxylique correspondant de formule :

$$Y\text{-}X\text{-}COOR \qquad\qquad (II)$$

dans laquelle R et X ont les mêmes significations que ci-dessus, et Y désigne un atome de chlore ou de brome, en milieu alcoolique anhydre ou ne contenant pas d'eau autre que celle pouvant provenir de l'éventuelle formation in situ du sulfhydrate, caractérisé en ce qu'on effectue la réaction sous une pression d'hydrogène sulfuré au moins égale à 10 bars absolus

2. Procédé selon la revendication 1, dans lequel l'alcool est le méthanol ou l'isopropanol.

3. Procédé selon la revendication 1 ou 2, dans lequel la pression d'hydrogène sulfuré est comprise entre 10 et 20 bars absolus.

4. , Procédé selon l'une des revendications 1 à 3, dans lequel on opère à une température allant de 0 à 80°C, de préférence comprise entre 10 et 60°C.

5. Procédé selon l'une des revendications 1 à 4, dans lequel la concentration initiale d'ester (II) dans le milieu réactionnel est comprise entre 1 et 5 moles par litre d'alcool.

6. Procédé selon l'une des revendications 1 à 5, dans lequel le rapport molaire sulfhydrate/ester (II) est compris entre 1 et 2, de préférence entre 1,1 et 1,5.

7. Procédé selon l'une des revendications 1 à 6, dans lequel on utilise le sulfhydrate d'ammonium.

**Patentansprüche**

1. Verfahren zur Herstellung eines Mercaptocarbonsäureesters der Formel (I)

$$HS\text{-}X\text{-}COOR \qquad\qquad (I)$$

in der X einen lineraren oder verzweigten Alkylenrest mit 1 bis 4 Kohlenstoffatomen und R einen linearen oder verzweigten Alkylrest mit 1 bis 18 Kohlenstoffatomen darstellt, durch Einwirkung von Ammoniumsulfhydrat oder Alkali- oder Erdalkalisulfhydrat auf den Halogencarbonsäureester der Formel (II)

$$Y\text{-}X\text{-}COOR \qquad\qquad (II)$$

in der R und X dieselbe Bedeutung wie zuvor haben und Y ein Chlor- oder Bromatom bezeichnet, in einem alkoholischen Milieu, das wasserfrei ist oder kein weiteres Wasser als dasjenige enthält, welches gegebenenfalls aus der in-situ-Bildung des Sulfhydrats stammt, dadurch gekennzeichnet, daß man die Reaktion unter einem Schwefelwasserstoffdruck von mindestens 10 bar absolut durchführt.

2. Verfahren nach Anspruch 1, bei dem der Alkohol Methanol oder Isopropanol ist.

3. Verfahren nach Anspruch 1 oder 2, bei dem der Schwefelwasserstoffdruck zwischen 10 und 20 bar absolut liegt.

**4.** Verfahren nach einem der Ansprüche 1 bis 3, bei dem man bei einer Temperatur von 0 °C bis 80 °C, vorzugsweise bei einer Temperatur von 10 °C bis 60 °C, verfährt.

**5.** Verfahren nach einem der Ansprüche 1 bis 4, bei dem die Anfangskonzentration an Ester (II) in dem Reaktionsmilieu zwischen 1 und 5 Mol pro Liter Alkohol liegt.

**6.** Verfahren nach einem der Ansprüche 1 bis 5, bei dem das Molverhältnis von Sulfhydrat/Ester (II) zwischen 1 und 2, vorzugsweise zwischen 1,1 und 1,5, liegt.

**7.** Verfahren nach einem der Ansprüche 1 bis 6, bei dem man Ammoniumsulfhydrat verwendet.

**Claims**

**1.** Process for the preparation of an ester of mercaptocarboxylic acid of formula:

$$HS-X-COOR \qquad\qquad (I)$$

in which X represents a linear or branched alkylene radical containing from 1 to 4 carbon atoms and R a linear or branched alkyl radical containing from 1 to 18 carbon atoms, by the action of ammonium hydrosulphide or of an alkali or alkaline-earth metal hydrosulphide on the corresponding halocarboxylic ester of formula:

$$Y-X-COOR \qquad\qquad (II)$$

in which R and X have the same meanings as above and Y denotes a chlorine or bromine atom, in anhydrous alcoholic medium or in a medium containing no water other than that which might arise from the possible formation in situ of the hydrosulphide, characterized in that the reaction is performed at a hydrogen sulphide pressure of at least 10 bars absolute.

**2.** Process according to Claim 1, in which the alcohol is methanol or isopropanol.

**3.** Process according to Claim 1 or 2, in which the hydrogen sulphide pressure is between 10 and 20 bars absolute.

**4.** Process according to one of Claims 1 to 3, in which the operation is carried out at a temperature ranging from 0 to 80°C, preferably between 10 and 60°C.

**5.** Process according to one of Claims 1 to 4, in which the initial concentration of ester (II) in the reaction medium is between 1 and 5 moles per litre of alcohol.

**6.** Process according to one of Claims 1 to 5, in which the hydrosulphide/ester (II) molar ratio is between 1 and 2, preferably between 1.1 and 1.5.

**7.** Process according to one of Claims 1 to 6, in which ammonium hydrosulphide is employed.